# EUROPEAN PATENT SPECIFICATION

(11) **EP 2 959 833 B1**
(45) Date of publication and mention of the grant of the patent: **05.09.2018**
(21) Application number: 14753573.6
(22) Date of filing: 20.02.2014
(51) Int. Cl.: A61B 5/087, A63B 23/18, A61B 5/00, A61B 5/08, A61B 5/09, A61B 7/04, A61B 7/00, A61M 16/00, A61M 16/20

(54) **RESPIRATORY EXERCISING DEVICE**
ATMUNGSÜBUNGSVORRICHTUNG
DISPOSITIF D'ENTRAÎNEMENT RESPIRATOIRE

(30) Priority: 20.02.2013 ES 201330226 P
(43) Date of publication of application: 30.12.2015
(73) Proprietor: Hospital Sant Joan de Deu, 08950 Esplugues de Llobregat Barcelona (ES)
(72) Inventor: RODRÍGUEZ NIEVA, Natalia, E-08950 Esplugues de Llobregat (Barcelona) (ES); CABRÉ MARTÍNEZ, Jordi, E-08950 Esplugues de Llobregat (Barcelona) (ES); DEL CAMPO GARCÍA RAMOS, Enrique, E-08950 Esplugues de Llobregat (Barcelona) (ES); FEBRER ROTGER, Anna, E-08950 Esplugues de Llobregat (Barcelona) (ES); ALONSO MERINO, Angel, E-08950 Esplugues de Llobregat (Barcelona) (ES)
(74) Representative: Carpintero Lopez, Francisco
(86) International application number: PCT/ES2014/070126
(87) International publication number: WO 2014/128331

(56) References cited:
- EP-A1- 2 283 773
- WO-A2-2012/038903
- WO-A2-2012/047792
- ES-T3- 2 081 101
- JP-A- H0 814 958
- US-A1- 2012 075 462
- US-A1- 2012 203 128

## Description

The present invention relates to a device for respiratory exercise which allows the measuring of the air flow and the monitoring of different exercises.

### Background of the invention

Respiratory physiotherapy is indicated as a coadjuvant treatment for many respiratory diseases with the following objectives: maintain the airways free from of secretions, achieve good mobility of the thoracic cavity and maintain good functioning of the respiratory muscles.

Respiratory processes, which are present with an increase of secretions, can cause bronchial obstruction if they are not mobilized with subsequent bacterial superinfection and possible pulmonary involvement.

Respiratory physiotherapy is composed of a series of respiratory exercises which achieve the objective of cleaning the airway, providing the arrival of air to all the possible spaces, mobilizing and expelling the secretions which had become stuck and facilitating such that the air can enter and exit from the airway without any problems. All of which is going to definitively lead to preventing or stopping a possible deterioration of the pulmonary function.

One of the principal problems which conventional respiratory physiotherapy for children poses is its difficulty in creating adherence in the form of a habit, due to the fact that the child ends up becoming tired of always practicing the same exercises.

Therefore, the need for a device is evident which allows for a child to utilize it as a game or for an adult to comfortably utilize it, the air flow being able to be readily detected and the need for a method which allows suitable monitoring.

In addition, devices are known which comprise a microphone which detect respiratory sounds during sleep, which are subsequently analyzed in order to detect possible sleep disruptions. However, these currently known devices are only geared to detect sleep disruptions, but not to be used as respiratory exercise, in particular in children.

WO2012038903A discloses a system for measuring spirometric flow rate using a microphone and a mobile computer device connected to or integrated with the microphone. The system has a transducer adapted for converting spirometric flow rate into an audible signal with an audio frequency characteristic of the spirometric flow rate.

### Description of the invention

Using the device and the method of the invention, the drawbacks cited can be solved, presenting other advantages which are described below.

The invention relates to a device for respiratory exercise, which comprises means for detecting the respiration of a person, and said means for detecting the respiration of a person comprise a turbine whistle which generates a variable frequency sound as a function of the air flow which passes through the same, and a microphone which captures said variable frequency sound generated by the turbine whistle.

Advantageously the respiratory exercise device of the present invention also comprises a filter associated with said microphone.

The respiratory exercise device also comprises an adjustable valve inserted at one air inlet end.

Furthermore, advantageously, the said microphone comprises means for sending the data of the captured sound generated by the turbine whistle, said means being, for example an antenna, wireless means or a connector.

The respiratory exercise device of the present invention preferably comprises an elongated hollow body, the microphone being located outside of said body and said turbine whistle being located in the interior of said body.

The method for respiratory exercise which said device comprises the steps of:
- obtaining data relating to the respiration of a person with the previously indicated device;
- sending said data relating to the respiration to a computer; and
- applying an algorithm to said data to calculate the values of the flows generated by the respiration.

Advantageously the method for respiratory exercise also comprises the step of storing the values of the flow generated by the respiration for the monitoring thereof.

Preferably the method for respiratory exercise of the present invention also comprises the step of converting the values of the flows generated by the respiration into real-time moving images, for example into a video game suitable for respiratory exercise in children or in adults.

According to a preferred embodiment, in the application of said algorithm the zero-cross rate (ZCR) is calculated and a zero-cross rate conversion table is applied to the flow.

Using the device of the present invention, respiratory exercise is achieved in a simple manner, even for children, using a simple device and a method which allows the monitoring on the part of a doctor and furthermore allows the respiratory exercise to be converted into a video game which incentivizes the use thereof in children and in adults.

### Brief description of the drawings

For a better understanding of what has been stated, drawings are enclosed, in which, schematically and only as a non-limiting example, a practical exemplary embodiment is depicted:
Figure 1 is a sectional schematic lateral view of the device of the present invention according to a first embodiment; and
Figure 2 is a sectional schematic lateral view of the device of the present invention according to a second embodiment.

### Description of a preferred embodiment

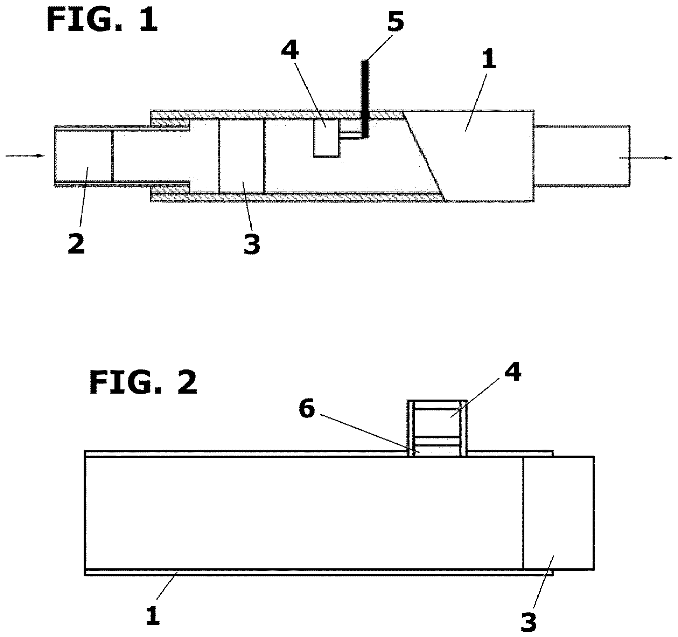

The respiratory exercise device of the present invention is formed by an elongated hollow body 1 which defines an air inlet end and an air outlet end and which comprises an adjustable valve 2 located at the air inlet end, a turbine whistle 3 and a microphone 4 as can be observed in figure 1, in a first embodiment.

A turbine whistle 3 is a whistle which generates a variable frequency sound as a function of the air flow which passes through the same such that the greater the flow that passes through, the greater the frequency.

Furthermore, the device of the present invention also comprises connection means for sending the data of the variable frequency sound generated by the turbine whistle 3. For example, said means are an antenna 5 connected to said microphone 4 which sends the data relating to the sound detected, such as the frequency, to a computer (not shown) or any suitable platform. In order to not cause turbulences, said microphone is associated with a filter 6.

It should be indicated that said connection means can also be any suitable means, such as wireless means (Wi-Fi, Bluetooth®, etc.) or any type of connector (mini-jack, USB, etc.).

In this way, when the user blows through the air inlet end, the air flow causes the whistle turbine 3 to generate a variable frequency sound which is captured by the microphone 4 which sends the data relating to said frequency to a computer or any suitable platform.

In figure 2 a second embodiment of the device of the present invention is depicted. It should be indicated that for reasons of simplicity, in figure 2, only the elements of the device inserted differently with respect to the first embodiment are shown.

In particular, the principal difference is the arrangement of the microphone 4 outside of said elongated hollow body 1 to prevent turbulences affecting said microphone.

The respiratory exercise method with said device comprises the following steps:
- when a user inhales or exhales into the previously indicated device, data relating to the respiration is generated, in particular frequency values;
- said device sends the frequency values to control means, such as a computer;
- in said control means, said frequency values pass through a conversion algorithm in which the zero-cross rate (ZCR) is detected and a zero-cross rate conversion table is applied to the flow, preferably in l/m; and
- said information is stored for the subsequent monitoring thereof on the part of a doctor.

Furthermore, said method can also comprise an additional step of converting said information regarding the respiration flow into real-time moving images, for example a video game such that a child or an adult can play at the same time as carrying out the suitable respiratory exercises.

## Claims

1. A respiratory exercise device comprising means for detecting the respiration of a person, said means for detecting the respiration of a person comprising a turbine whistle (3) which generates a variable frequency sound as a function of the air flow which passes through the same and a microphone (4) which captures said variable frequency sound generated by the turbine whistle (3), **characterized in that** the device also comprises an adjustable valve (2) inserted at an air inlet end of the device.

2. The respiratory exercise device according to claim 1, wherein it also comprises a filter (6) associated with said microphone (4).

3. The respiratory exercise device according to claim 1, wherein it comprises an elongated hollow body (1), said microphone (4) being located outside of said body and said turbine whistle (3) being located in the interior of said body.

4. The respiratory exercise device according to claim 1, wherein said microphone (4) comprises connection means for sending the data of the variable frequency sound generated by the turbine whistle (3).

5. The respiratory exercise device according to claim 4, wherein said means for sending the data of the variable frequency sound generated by the turbine whistle (3) comprises an antenna (5), a wireless connection or a connector.

## Patentansprüche

1. Atemübungs-Vorrichtung, die Einrichtungen zum Erfassen der Atmung einer Person umfasst, wobei die Einrichtungen zum Erfassen der Atmung einer Person eine Turbinen-Pfeife (3), die in Abhängigkeit von dem Luftstrom durch sie hindurch einen Ton variabler Frequenz erzeugt, sowie ein Mikrofon (4) umfassen, das den durch die Turbinen-Pfeife (3) erzeugten Ton variabler Frequenz erfasst, **dadurch gekennzeichnet, dass** die Vorrichtung des Weiteren ein verstellbares Ventil (2) umfasst, das in ein Lufteinlass-Ende der Vorrichtung eingeführt ist.

2. Atemübungs-Vorrichtung nach Anspruch 1, wobei sie des Weiteren einen mit dem Mikrofon (4) verbundenen Filter (6) umfasst.

3. Atemübungs-Vorrichtung nach Anspruch 1, wobei sie einen länglichen hohlen Körper (1) umfasst, sich das Mikrofon (4) außerhalb des Körpers befindet und sich die Turbinen-Pfeife (3) im Inneren des Körpers befindet.

4. Atemübungs-Vorrichtung nach Anspruch 1, wobei das Mikrofon (4) eine Verbindungseinrichtung umfasst, über die die Daten des durch die Turbinen-Pfeife (3) erzeugten Tons variabler Frequenz gesendet werden.

5. Atemübungs-Vorrichtung nach Anspruch 4, wobei die Einrichtung über die die Daten des durch die Turbinen-Pfeife (3) erzeugten Tons variabler Frequenz gesendet werden, eine Antenne (5), eine Drahtlos-Verbindung oder einen Verbinder umfasst.

## Revendications

1. Appareil d'exercice respiratoire comprenant un moyen pour détecter la respiration d'une personne, ledit moyen pour détecter la respiration d'une personne comprenant un sifflet de turbine (3) qui produit un son de fréquence variable en fonction du flux d'air qui la traverse et un microphone (4) qui capture ledit son de fréquence variable produit par le sifflet de turbine (3), **caractérisé en ce que** le dispositif comprend également une soupape réglable (2) insérée au niveau d'une extrémité d'entrée d'air du dispositif.

2. Appareil d'exercice respiratoire selon la revendication 1, dans lequel il comprend également un filtre (6) associé audit microphone (4).

3. Appareil d'exercice respiratoire selon la revendication 1, dans lequel il comprend un corps creux allongé (1), ledit microphone (4) étant situé à l'extérieur dudit corps et ledit sifflet de turbine (3) étant situé à l'intérieur dudit corps.

4. Appareil d'exercice respiratoire selon la revendication 1, dans lequel ledit microphone (4) comprend un moyen de connexion pour envoyer les données du son de fréquence variable produit par le sifflet de turbine (3).

5. Appareil d'exercice respiratoire selon la revendication 4, dans lequel ledit moyen pour envoyer les données du son de fréquence variable produit par le sifflet de turbine (3) comprend une antenne (5), une connexion sans fil ou un connecteur.
